(19) Europäisches Patentamt
European Patent Office
Office européen des brevets

(11) Publication number: **0 394 542 A1**

# (12) EUROPEAN PATENT APPLICATION

(21) Application number: 89107999.8

(22) Date of filing: 03.05.89

(51) Int. Cl.5: A61K 31/135, A61K 31/275, A61K 31/41, A61K 31/165

(30) Priority: 25.04.89 US 341351

(43) Date of publication of application: 31.10.90 Bulletin 90/44

(84) Designated Contracting States: AT BE CH DE ES FR GB GR IT LI NL SE

(71) Applicant: AMERICAN CYANAMID COMPANY
1937 West Main Street P.O. Box 60
Stamford Connecticut 06904-0060(US)

(72) Inventor: Ruszala-Mallon, Veronica M.
395 North Little Tor Road
New City New York 10956(US)
Inventor: Wallace, Roslyn E.
27 North Pearl Street
Pearl River New York 10965(US)
Inventor: Wang, Bosco Shang
10 Parkview Road
Cranbury New Jersey 08512(US)
Inventor: Lin, Yang-I
35 Constitution Drive
Tappan New York(US)

(74) Representative: Wächtershäuser, Günter, Dr.
Tal 29
D-8000 München 2(DE)

## (54) Bone marrow cell recovery with synthetic csf inducers.

(57) The invention is a method of accelerating recovery of bone marrow stem cells and/or progenitor cells by the administration of N-substituted phenyl-thioanilines, N-substituted-phenylsulfinylanilines, and N-substituted-phenylsulfonylanilines.

EP 0 394 542 A1

Xerox Copy Centre

# BONE MARROW CELL RECOVERY WITH SYNTHETIC COLONY STIMULATING FACTOR (CSF) INDUCERS

## SUMMARY OF THE INVENTION

The invention is the use of N-substituted-phenylthioanilines, N-substituted-phenylsulfinylanilines and N-substituted-phenylsulfonylanilines for enhancing bone marrow cell recovery in warm-blooded animals in need of such enhancement.

## DESCRIPTION OF THE DRAWINGS

Figure 1 - A Bar Chart of the Dose Response of N-[4-[(4-fluorophenyl)sulfonyl]phenyl]acetamide on CSF Production.

Figure 2 - A Bar Chart of the Effect of N-[4-[(4-fluorophenyl)sulfonyl]phenyl]acetamide on the Recovery of 5-FU Induced Myelosuppression in Mice.

Figure 3 - A Bar Chart of the Effect of N-[4-[(4-fluorophenyl)sulfonyl]phenyl]acetamide on the Recovery of Bone Marrow CFU-C Following Ara-Ac Treatment.

Figure 4 - A Bar Chart of the Effect of N-[4-[(4-fluorophenyl)sulfonyl]phenyl]acetamide on the Recovery of Bone Marrow CFU-C Following Cyclophosphamide Treatment.

Figure 5 - A Bar Chart of the Effect of N-[4-[(fluorophenyl)sulfonyl]phenyl]acetamide on the Recovery of 5-FU Induced Neutropenia in Athymic Mice.

Figure 6 - A Bar Chart of the Effect of N-[4-[(4-fluorophenyl)sulfonyl]phenyl]acetamide on Platelet Counts in 5-FU Treated Mice.

Figure 7 - A Bar Chart of the Effect of N-[4-[(4-fluorophenyl)sulfonyl]phenyl]acetamide on Reticulocyte Counts in 5-FU Treated Mice.

Figure 8 - A Bar Chart of the Effect of N-[4-[(4-fluorophenyl)sulfonyl]phenyl]acetamide on Bone Marrow Cell Recovery After Gamma Irradiation.

Figure 9 - A Bar Chart of the Effect of N-[4-[(4-fluorophenyl)sulfonyl]phenyl]acetamide on Endogenous CFU-S Following 600 Rads of Gamma Irradiation.

Figure 10 - A Bar Chart of the Enhancement of IL-1 Production.

## DESCRIPTION OF THE INVENTION

The invention is a method of enhancing bone marrow cell recovery in a warm-blooded animal which comprises administering to the animals an effective amount of a compound of the formula:

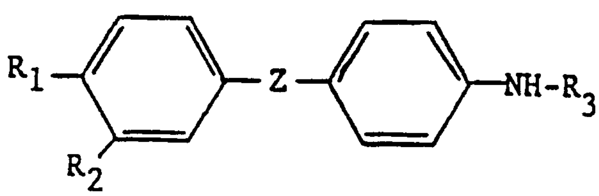

wherein $R_1$ is hydrogen, fluoro, chloro, bromo, nitro, alkoxy having up to 3 carbon atoms or $-NHCOCH_2NHCH_3$; $R_2$ is hydrogen or chloro; and $R_3$ is $-CH_2CN$, $-COCH_2NH_2$, $-COCH_2NHCH_3$, $-COCH_2Cl$, $-COCH_2CH_2Cl$,

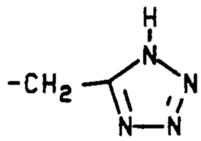

or

O
II
-C -R

wherein R is alkyl having up to 4 carbon atoms such as methyl, isopropyl, n-butyl, tert-butyl, etc.; and Z is thio (-S-), sulfinyl (-SO-) or sulfonyl ($-SO_2-$); with the proviso that at least one of $R_1$ and $R_2$ must be hydrogen but $R_1$ and $R_2$ may not both be hydrogen; together with the pharmaceutically acceptable salts thereof; in a pharmaceutically effective amount.

The compounds useful in our invention may be prepared in accordance with the following reaction scheme as taught in U.S. Patent 4,532,349:

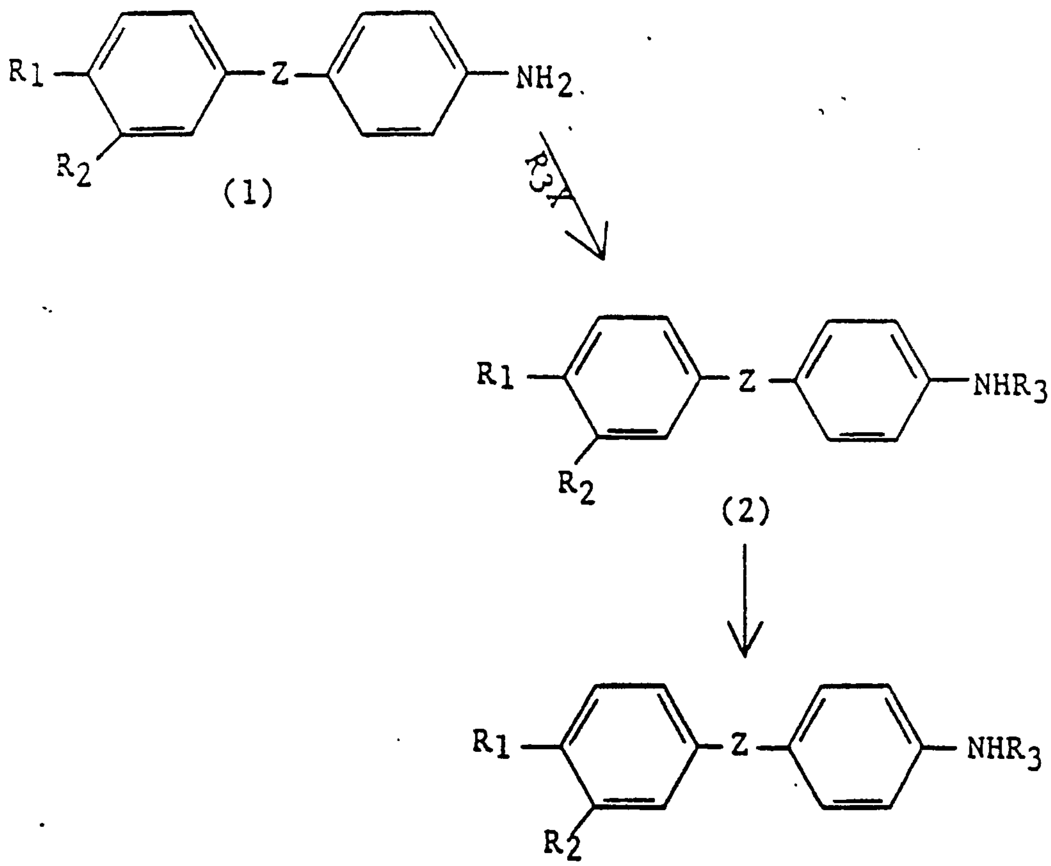


(1)

(2)

(3)

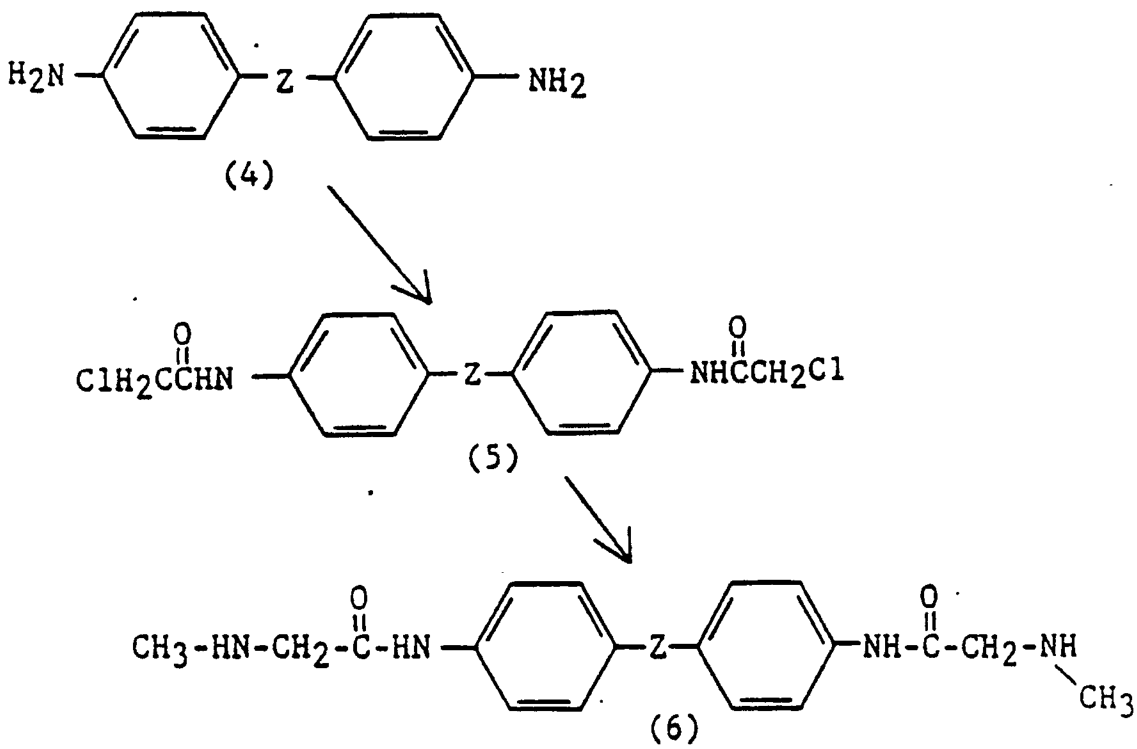

(4)

(5)

(6)

wherein $R_1$, $R_2$, $R_3$ and Z are as hereinabove defined. In accordance with the above reaction scheme, a 4-amino-substituted phenyl sulfide, sulfoxide or sulfone (1) is reacted with $R_3X$ where $R_3$ is $-COCH_2-CH_2-Cl$, $-COCH_2Cl$, $-CH_2CN$ or -COR and X is chloro or bromo in a solvent such as toluene at reflux for several hours. The solvent is evaporated and the product crystallized from a solvent such as ethanol giving (2). The compounds (2), where $R_3$ is $-COCH_2Cl$, may then be reacted with methylamine or ammonia to give the products (3) where $R_3$ is $-COCH_2NH_2$ or $-COCH_2NHCH_3$. The compounds (2) where $R_2$ is $-COCH_2Cl$ may also be treated with sodium azide to give azido derivatives which are then reduced with hydrogen sulfide and triethylamine or hydrogen and Raney nickel to give the products (3) where $R_3$ is $-COCH_2NH_2$. The compounds (2) where $R_3$ is $-CH_2CN$ may be reacted with sodium azide to give the products (3) where $R_3$ is

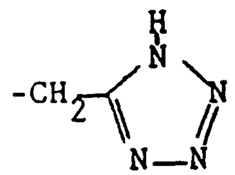

A bis(p-aminophenyl)sulfide, sulfoxide or sulfone (4) where Z is -S-, -SO- or $-SO_2$ is reacted with chloroacetyl chloride to give a bis[p-chloroacetamido)phenyl]sulfide or sulfoxide or sulfone (5) which is then reacted with methylamine in ethanol at reflux for several hours giving (6) where Z is as described above.

Modern research is directed to the discovery of a drug similar to, but more potent than, known immunoregulants such as levamisole that would be effective in the eradication of tumor cells when used in conjunction with standard therapeutic measures. Stimulators of host resistance may be detected in animal models that can, in fact, detect both immuno-stimulators and anticancer agents. Mice are put in a condition simulating immunodepression common to cancer patients. This is accomplished by treating mice with chemotherapeutic drugs or with gamma irradiation. A major side effect associated with such therapy is myelosuppression which then limits the dose of drug used and/or the frequency of treatment. Deaths due to chemo or irradiation associated myelosuppression are generally due to hemorrhage or sepsis. The hemorrhagic deaths are a result of thrombocytopenia, a drastic decrease in the number of platelets. Septic deaths are a result of neutropenia, a severe drop in neutrophils, the cell which plays a major role in recovery from bacterial infections. Septic deaths occur even when patients are treated with antibiotics.

However, it is known that with many cancers, the outcome may be better if one could use a more aggressive therapeutic approach by either increasing the dose or frequency of chemo- or radiotherapy. Thus, a method for protecting the bone marrow from cytotoxic agents or accelerating the recovery of bone marrow cells following these regimens may allow for such aggressive therapy. One such approach to overcoming the hematologic toxicity associated with cancer therapies is autologous bone marrow transplantation to accelerate recovery of hematopoietic cells. More recently several factors have been identified which are known stimulators of hematopoietic cell growth. These colony stimulating factors (CSFs) act on a variety of bone marrow progenitor cells to accelerate their differentiation into mature, active cell populations. Several of these factors have been cloned and are currently in trials in cancer patients who have undergone a variety of chemotherapeutic regimens. Results show that these agents, in particular, GCSF (granulocyte colony stimulating factor) and GM-CSF (granulocyte macrophage colony stimulating factor), could stimulate a sustained rise in neutrophil counts thus reducing the period of neutropenia after administration of a cytotoxic agent. A reduction in the number of days of neutropenia may prove beneficial ultimately in alleviating the morbidity and mortality associated with cancer therapy and also in terms of hospital costs (by reducing the need for inpatient care).

However, there are several drawbacks to treatment of chemotherapy associated myelosuppression with the recombinant colony stimulating factors. For one, there have been studies which indicate toxicities associated with GM-CSF therapy itself. Secondly, since the recombinant products are protein in nature, they have relatively short half life when given intravenously. Thus, there is a need for continuous I.V. infusion to maintain therapeutically active levels. Thirdly, current studies are pointing to the need for combination therapy with various cytokines in order to obtain maximal responsiveness in bone marrow cell recovery. Compounds such as the one described in this invention have several advantages over the recombinant CSFs. The primary advantage lies in the oral effectiveness of our invention thus eliminating the need for continuous I.V. infusion and longer hospital stays. Secondly, the cost of manufacturing a synthetic compound is much less than that of a recombinant protein, thus translating into reduced cost for the patient. Thirdly, orally active synthetic compounds such as the one described here are very stable compared with the recombinant proteins which are easily degraded once administered I.V. Thus, a single oral dose of the

synthetic compounds results in therapeutic effects comparable to a 14 day continuous infusion regimen with the recombinant CSFs. And finally, one additional advantage of the synthetic compound over the CSFs lies in the multiple cytokine induction by the synthetics, in particular, IL-1 and CSF. One can achieve in a single oral dose with the synthetic compounds what multiple cytokines may do with repeated dosing.

The compounds useful in our invention are an alternative to the CSFs to allow more aggressive therapy with cytotoxic drugs whose dose limiting toxicity is severe myelosuppression. Effective drugs such as the ones described here are recognized by their ability to accelerate recovery of bone marrow colony forming cells following chemotherapy and by stimulation of a colony forming activity in the serum of mice which allows for the proliferation of normal bone marrow cells in culture. A compound's radioprotective property is measured by its ability to enhance the number of colony forming cells in the spleen of treated mice (endogenous CFUs). Its ability to augment production of IL-1, a cytokine which acts synergistically with the CSFs, is measured by the ability of macrophage supernatants of treated mice to promote growth of thymocytes in culture.

The invention is especially useful for stimulating the proliferation and differentiation of blood cell progenitors in bone marrow of warm-blooded animals, accelerating the recovery of white blood cell progenitors in bone marrow of warm-blooded animals, enhancing the activity of immune cells which inhibit tumor growth in warm-blooded animals, enhancing the activity of immunoregulatory proteins in warm-blooded animals, enhancing the activity of immune cells and immunoregulatory proteins which inhibit bacterial and viral growth in warm-blooded animals, and increasing the percentage of red blood cell progenitors in warm-blooded animals. The invention includes the use of the synthetic CSF inducers herein to manufacture medicaments for these uses.

The compounds useful in our invention are active as synthetic CSF inducers when tested according to the following procedures:

## Induction of Colony Stimulating Factor(s)

Mice were treated orally with N-[4-[(4-fluorophenyl)sulfonyl]phenyl]acetamide on day zero at doses of 12.5, 25, 50, 100, 200, or 400 mg/kg. Four days later the mice were bled retroorbitally and the serum tested for CSF activity on cultures of normal bone marrow cells. Five $x10^4$ normal bone marrow cells were plated in agarose containing medium supplemented with 10% serum from either normal mice or mice treated with N-[4-[(4-fluorophenyl)sulfonyl]phenyl]acetamide. A positive control consisted of cells cultured with an exogenous source of CSF with known activity (either L cell conditioned medium or recombinant GM-CSF). Cultures were incubated for 7 days at 37°C and the number of colonies consisting of 50 or more cells were counted. By definition, the net formation of one colony above background equals 1 unit of CSF activity. As shown in Figure 1 serum from mice given one oral dose of N-[4-[(4-fluorophenyl)sulfonyl]phenyl]acetamide contained significantly higher levels of CSF than normals. Optimal doses for induction of CSF ranged from 50 to 200 mg/kg.

## Acceleration of Myeloid Cells in the Bone Marrow of Mice Treated with Cytoreductive Therapy

Groups of mice were treated I.P. with either 5-FU (150 mg/kg), cyclophosphamide(100 mg/kgx3), or ARA-AC(800 mg/kg). Four days later they were given one oral dose of synthetic CSF inducer at 25, 100, or 200 mg/kg. Three days later the mice were sacrificed and bone marrow cells collected by aspiration through a 23 gauge needle and dispensed into 5 ml of RPMI-1640 culture medium. Aliquots were detected with a 0.2% solution of acetic acid in PBS, containing 0.2% crystal violet. The number of nucleated cells per femur was determined by direct count in a hemocytometer. The cells were plated in agarose containing medium supplemented with an exogenous source of CSF (50 l/plate of GM-CSF). After 7 days in culture the number of colonies consisting of 50 or more cells are counted and the values expressed as CFU-C per 100,000 cells. The results are expressed as % of control.

As shown in Figure 2, there was significant acceleration of recovery of bone marrow colony forming cells after 5-FU when mice were treated with N-[4-[(4-fluorophenyl)sulfonyl]phenyl]acetamide on day 2 (when 100 mg/kg of N-[4-[(4-fluorophenyl)sulfonyl]phenyl]acetamide was given) or when given on day 4 after 5-FU at either 100 or 25 mg/kg.

Likewise, N-[4-[(4-fluorophenyl)sulfonyl]phenyl]acetamide at 100 mg/kg significantly accelerated recovery of bone marrow CFU-C when given on day 4 after ARA-AC (Figure 3) or cytoxan (Figure 4).

The effects of N-[4-[(4-fluorophenyl)sulfonyl]phenyl]acetamide on recovery of neutrophils following

chemotherapy induced neutropenia were monitored and the results shown in Figure 5. Mice treated with 5-FU have significantly depressed levels of neutrophils in the peripheral blood. However, when treated with N-[4-[(4-fluorophenyl)sulfonyl]phenyl]acetamide on day 4 following 5-FU there was significant recovery of this cell population compared to untreated controls. Platelet and reticulocyte counts were also restored to a moderate degree when mice received N-[4-[(4-fluorophenyl)sulfonyl]phenyl]acetamide after 5-FU therapy (Figures 6 and 7).

The effects of N-[4-[(4-fluorophenyl)sulfonyl]phenyl]acetamide on hematopoiesis are also seen in the spleen of treated mice (Table 1). By 4 to 9 days after treatment with N-[4-[(4-fluorophenyl)sulfonyl]phenyl]-acetamide single cell suspensions of splenocyte preparations reveal an increase in cells of predominantly the granulocytic series.

Table 1

| Effect of N [4-[(4-fluorophenyl)sulfonyl]phenyl]acetamide on differential cell counts in spleens of treated mice | | | | | | | | |
|---|---|---|---|---|---|---|---|---|
| | % of Total | | | | | | | |
| | Granulocytes | | | | | | | |
| Mouse Group[a] | M[b] | L | P | Rings | JU | MY | EO | X |
| Normals | 2 | 74 | 8 | 4 | 1 | 0 | 6 | 5 |
| N -[4-[(4-fluorophenyl)sulfonyl]phenyl]acetamide | | | | | | | | |
| 400 | 0 | 80 | 8 | 7 | 2 | 0 | 1 | 1 |
| 200 | 0 | 78 | 10 | 5 | 1 | 0 | 2 | 3 |
| 100 | 3 | 59 | 14 | 10 | 1 | 1 | 7 | 5 |
| 50 | 2 | 63 | 14 | 14 | 1 | 2 | 3 | 1 |

[a] Mice were sacrificed four days after treatment with N-[4-[(4-fluorophenyl)sulfonyl]phenyl]acetamide and splenocytes stained with Giemsa stain and cytospin preparations examined.
[b] Abbreviations: M, monocyte; L, lymphocyte; P, polymorphonuclear neutrophilic granulocyte; Rings, neutrophilic bands; JU, juvenile neutrophilic metamyelocyte; MY, neutrophilic myelocyte; EO, eosinoph l; X, unknowns.

## Radioprotection Studies

The radioprotective effect of N-[4-[(4-fluorophenyl)sulfonyl]phenyl]acetamide was measured in 2 ways:

## CFU-C Assay

Mice were treated with N-[4-[(4-fluorophenyl)sulfonyl]phenyl]acetamide at various times relative to 100 or 200 rads of whole body gamma irradiation. Four days after irradiation the mice were sacrificed and bone marrow cells collected and plated in an agarose containing culture with an exogenous source of CSF as described above. Seven days later the bone marrow colonies were counted and the results expressed as % of control. As shown in Figure 8 N-[4-[(4-fluorophenyl)sulfonyl]phenyl]acetamide was radioprotective when given on day -1, 0 or +1 relative to irradiation. However, optimal protection was observed when it was given one day prior to irradiation.

## Endogenous CFU-S Assay

Balb/c mice were treated with N-[4-[(4-fluorophenyl)sulfonyl]phenyl]acetamide one day prior to receiving high dose (600 rads) whole body irradiation. The control group received no drug prior to irradiation. Two

weeks later the mice were sacrificed and the spleen removed and fixed in Bouins fixative. The number of colonies per spleen is counted and is reflective of the number of bone narrow cells remaining after irradiation. As shown in Figure 9 N-[4-[(4-fluorophenyl)sulfonyl]phenyl]acetamide was radioprotective as evidenced by the significantly greater number of spleen colonies compared to the untreated control groups.

IL-1 Assay

Groups of C57B1/6 mice were treated orally with N-[4-[(4-fluorophenyl)sulfonyl]phenyl]acetamide 100 mg/kg. Four days later peritoneal exudate cells (PEC) were collected and 1 x $10^5$ cells were plated in RPMI-1640 medium containing 5% fetal calf serum (FCS). After 2 hours incubation at 37° C the non-adherent cells were washed off and the adherent cells were incubated for 24 hours in RPMI medium with 5% FCS with or without Lipopolysaccharide or phenyl myristic acetate. The following day the supernatants were collected and assayed for IL-1 on thymocytes. The cultures were incubated for 3 days and then pulsed with $^3$H-TdR using 0.5 ICi/well. The cells were harvested and the number of counts per minute (CPM) was determined using a Beckman scintillation counter. As shown in Figure 10, macrophages from N-[4-[4-fluorophenyl)sulfonyl]phenyl]acetamide treated mice release significantly greater levels of IL-1 than macrophages from untreated mice.

The compounds of our invention are effective as synthetic CSF inducers when administered orally in amounts ranging from about 5 mg. to about 400 mg. per kilogram of body weight per day. A preferred dosage regimen for optimum results would be from about 5 mg. to about 50 mg. per kilogram of body weight, and such dosage units are employed that a total of from about 350 mg. to about 3.5 grams of the active compound for a subject of about 70 kg. of body weight are administered in a 24 hour period. This dosage regimen may be adjusted to provide the optimum therapeutic response. For example, several divided doses may be administered daily or the dose may be proportionally reduced as indicated by the exigencies of the therapeutic situation. A practical advantage of our invention is that the active compounds may be administered in any convenient manner such as the oral or buccal routes.

The compounds of our invention may be orally administered, for example, with an inert diluent or with an assimilable edible carrier, or they may be enclosed in hard or soft shell gelatin capsules, or they may be compressed into tablets, for oral therapeutic administration, the active compounds may be incorporated with excipients and used in the form of tablets, troches, capsules, elixirs, suspensions, syrups, wafers, and the like. Such compositions and preparations should contain at least 0.5% of active compound. The percentage of the compositions and preparations may, of course, be varied and may conveniently be between about 2% to about 60% of the weight of the unit. The amount of active ingredient in such therapeutically useful compositions is such that a suitable dosage will be obtained. Preferred compositions or preparations according to the present invention are prepared so that an oral dosage unit form contains between about 50 and 250 milligrams of active compound. The tablets, troches, pills, capsules and the like may also contain the following: a binder such as gum tragacanth, acacia, corn starch or gelatin; excipients such as dicalcium phosphate; disintegrating agents such as corn starch, potato starch, alginic acid and the like; a lubricant such as magnesium stearate; and a sweetening agent such as sucrose, lactose or saccharin may be added or a flavoring agent such as peppermint, oil of wintergreen, or cherry flavoring. When the dosage unit form is a capsule, it may contain, in addition to materials of the above type, a liquid carrier such as a fatty oil. Various other material may be present as coatings or to otherwise modify the physical form of the dosage unit. For instance, tablets, pills, or capsules may be coated with shellac, sugar or both. A syrup or elixir may contain the active compound, sucrose as sweetening agent, methyl and propylparabens as preservatives, a dye and flavoring such as cherry or orange flavor. Of course, any material used in preparing any dosage unit form should be pharmaceutically pure and substantially non-toxic in the amounts employed.

## Claims

1. A method of enhancing bone marrow cell recovery in warm-blooded animals in need for such enhancement which comprises administering to such animals a therapeutically effective amount of a compound of the formula:

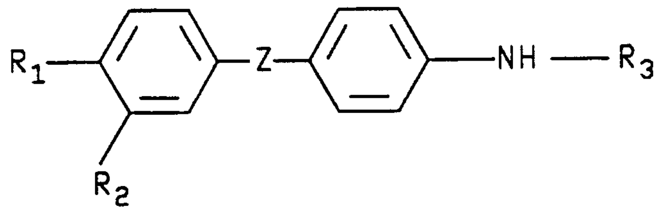

wherein $R_1$ is hydrogen, fluoro, chloro, bromo, nitro, alkoxy having up to 3 carbon atoms or $-NHCOCH_2NHCH_3$; $R_2$ is hydrogen or chloro; and $R_3$ is $-CH_2CN$, $-COCH_2NH_2$, $-COCH_2NHCH_3$, $-COCH_2Cl$, $-COCH_2CH_2Cl$,

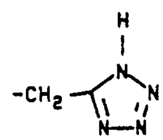

or

O
II
-C -R

wherein R is alkyl having up to 4 carbon atoms; and Z is thio (-S-), sulfinyl (-SO-) or sulfonyl ($-SO_2-$); with the proviso that at least one of $R_1$ and $R_2$ must be hydrogen but $R_1$ and $R_2$ may not both be hydrogen; or pharmaceutically acceptable salts thereof.

2. A method according to Claim 1 of stimulating the proliferation and differentiation of blood cell progenitors in bone marrow of warm-blooded animals in need of such stimulation.

3. A method according to Claim 1 of accelerating the recovery fo white blood cell progenitors in bone marrow of warm-blooded animals in conjunction with chemical or irradiation therapy.

4. A method according to Claim 1 of enhancing the activity of immune cells which inhibit tumor growth in warm-blooded animals having tumor growth and in need of such enhancement.

5. A method according to Claim 1 of enhancing the activity of immunoregulatory proteins which inhibit tumor growth in warm-blooded animals having tumor growth and in need of such enhancement.

6. A method according to Claim 1 of enhancing the activity of immune cells and immunoregulatory proteins which inhibit bacterial growth in warm-blooded animals having bacterial infection and in need of such enhancement.

7. A method according to Claim 1 of enhancing the activity of immune cells and immunoregulatory proteins which inhibit viral growth in warm-blooded animals having viral infection and in need of such enhancement.

8. A method according to Claim 1 for increasing the percentage of red blood projenitors in warm-blooded animals in need of such increase.

9. The use of a compound of the formula:

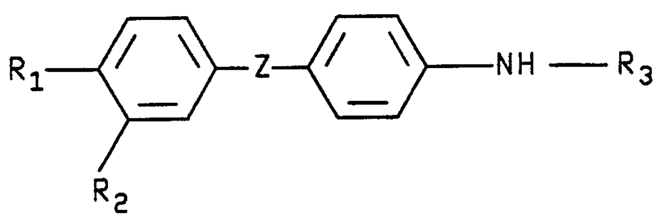

wherein $R_1$ is hydrogen, fluoro, chloro, bromo, nitro, alkoxy having up to 3 carbon atoms or $-NHCOCH_2NHCH_3$; $R_2$ is hydrogen or chloro; and $R_3$ is $-CH_2CN$, $-COCH_2NH_2$, $-COCH_2NHCH_3$, $-COCH_2Cl$, $-COCH_2CH_2Cl$,

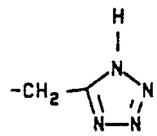

or

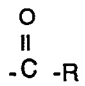

wherein R is alkyl having up to 4 carbon atoms; and Z is thio (-S-), sulfinyl (-SO-) or sulfonyl ($-SO_2-$); with the proviso that at least one of $R_1$ and $R_2$ must be hydrogen but $R_1$ and $R_2$ may not both be hydrogen; or pharmaceutically acceptable salts thereof for the manufacture of a medicament ready for the treatment of enhancing bone marrow cell recovery in warm-blooded animals in need of such enhancement.

10. The use according to Claim 9 for the manufacture of a medicament ready for the treatment of increasing the percentage of red blood cell projenitors in warm-blooded animals in need of such increase.

Figure 1

Dose Response of N-[4-[(4-Fluorophenyl)sulfonyl]phenyl]acetamide on CSF Production

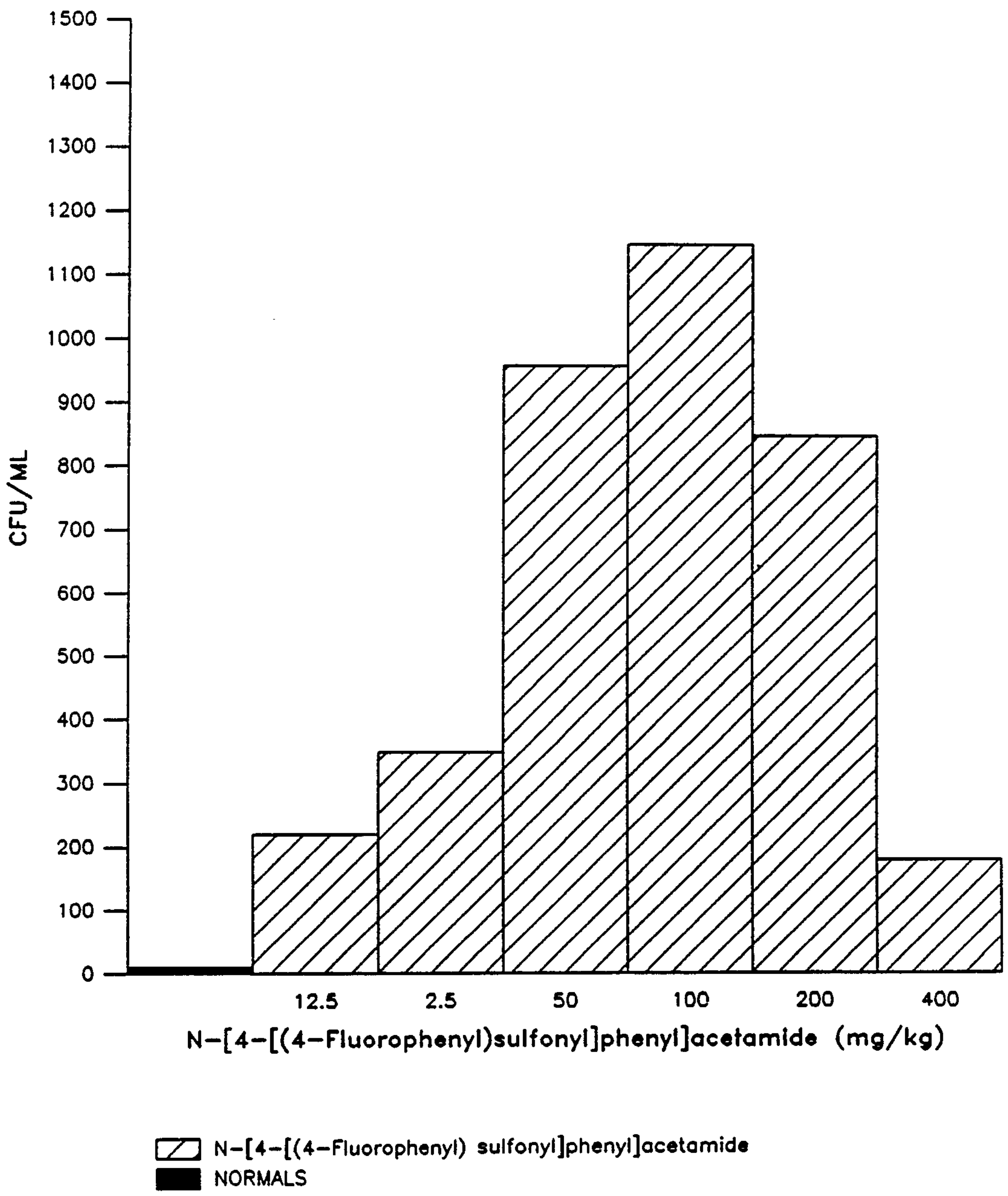

Figure 2

Effect of N-[4-[(4-Fluorophenyl)sulfonyl]phenyl]acetamide on Recovery of 5-FU Induced Myelosuppression in Mice

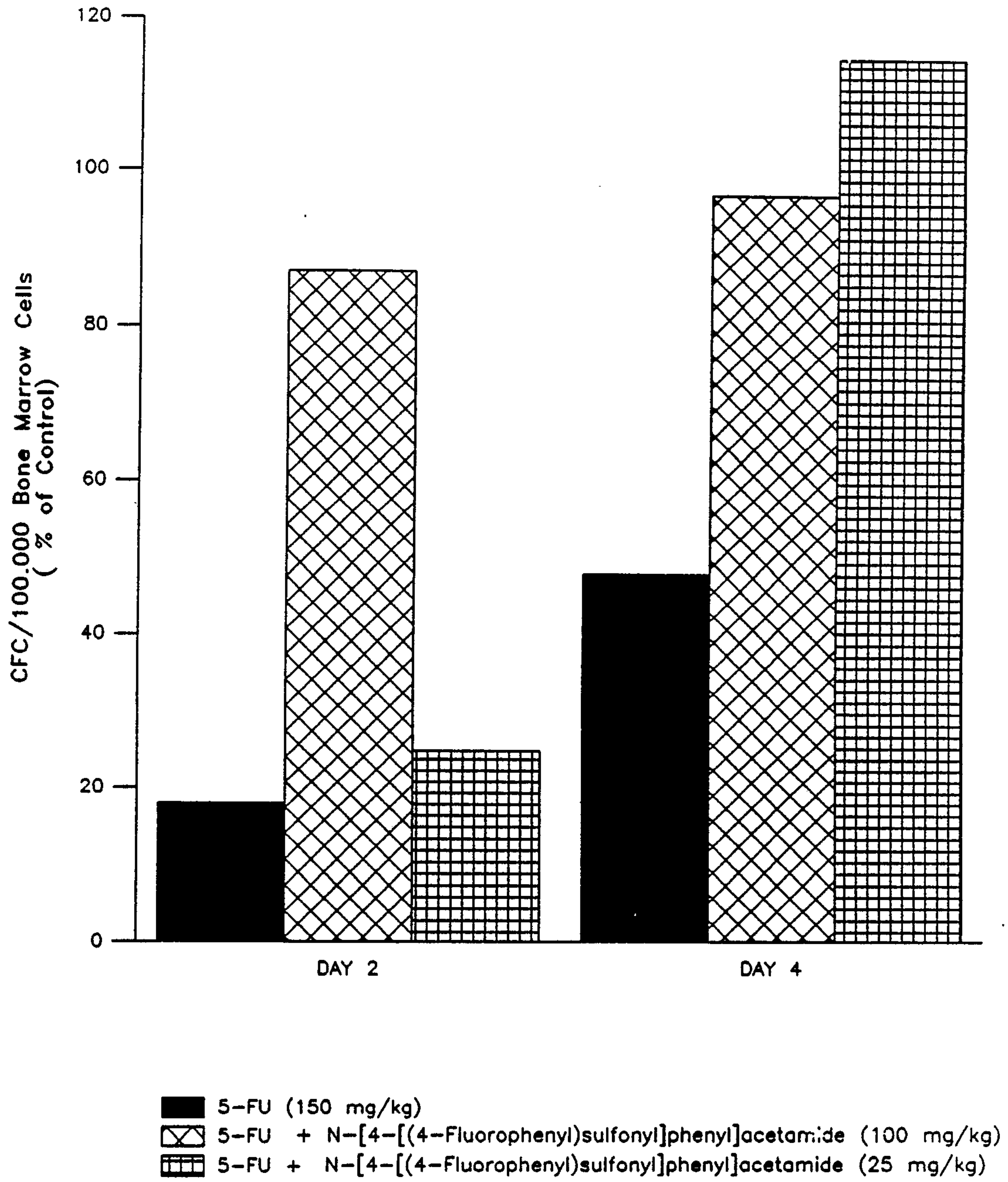

Figure 3

Effect of N-[4-[(4-Fluorophenyl)sulfonyl]phenyl]acetamide on Recovery of Bone Marrow CFU-C Following Ara-AC Treatment

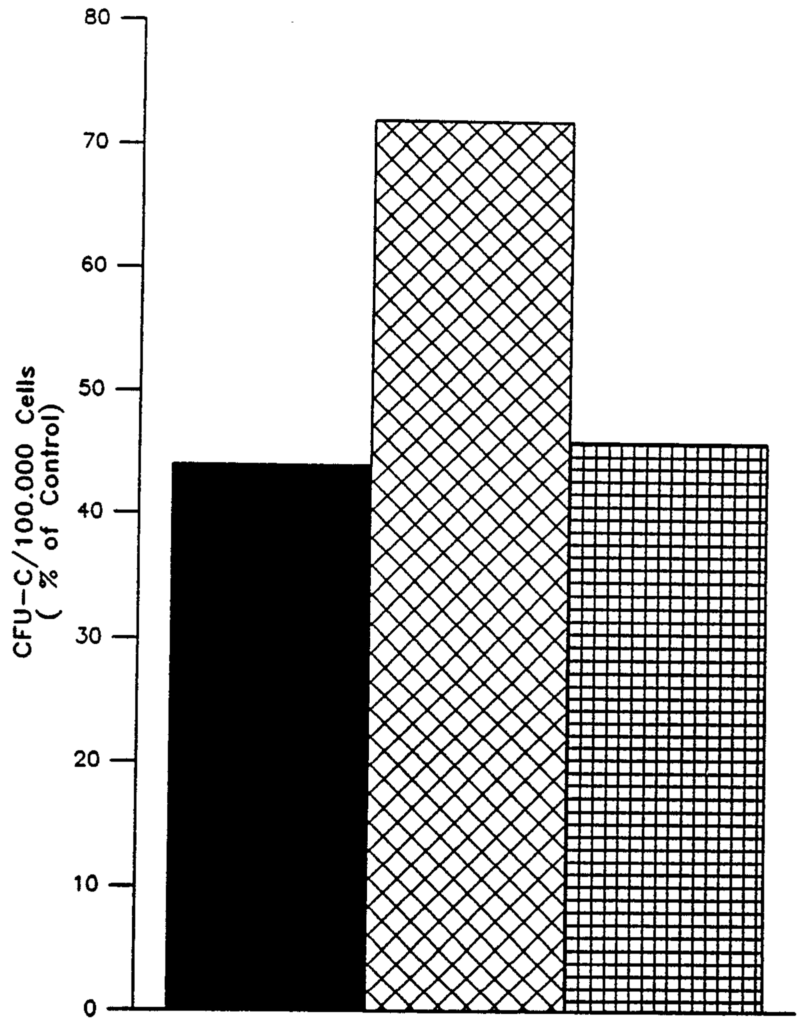


Ara-AC (800 mg/kg)
Ara-AC + N-[4-[(4-Fluorophenyl)sulfonyl]phenyl]acetamide (100 mg/kg)
Ara-AC + N-[4-[(4-Fluorophenyl)sulfonyl]phenyl]acetamide (25 mg/kg)

Figure 4

Effect of N-[4-[(4-Fluorophenyl)sulfonyl]phenyl]acetamide on Recovery of Bone Marrow CFU-C Following Cyclophosphamide Treatment

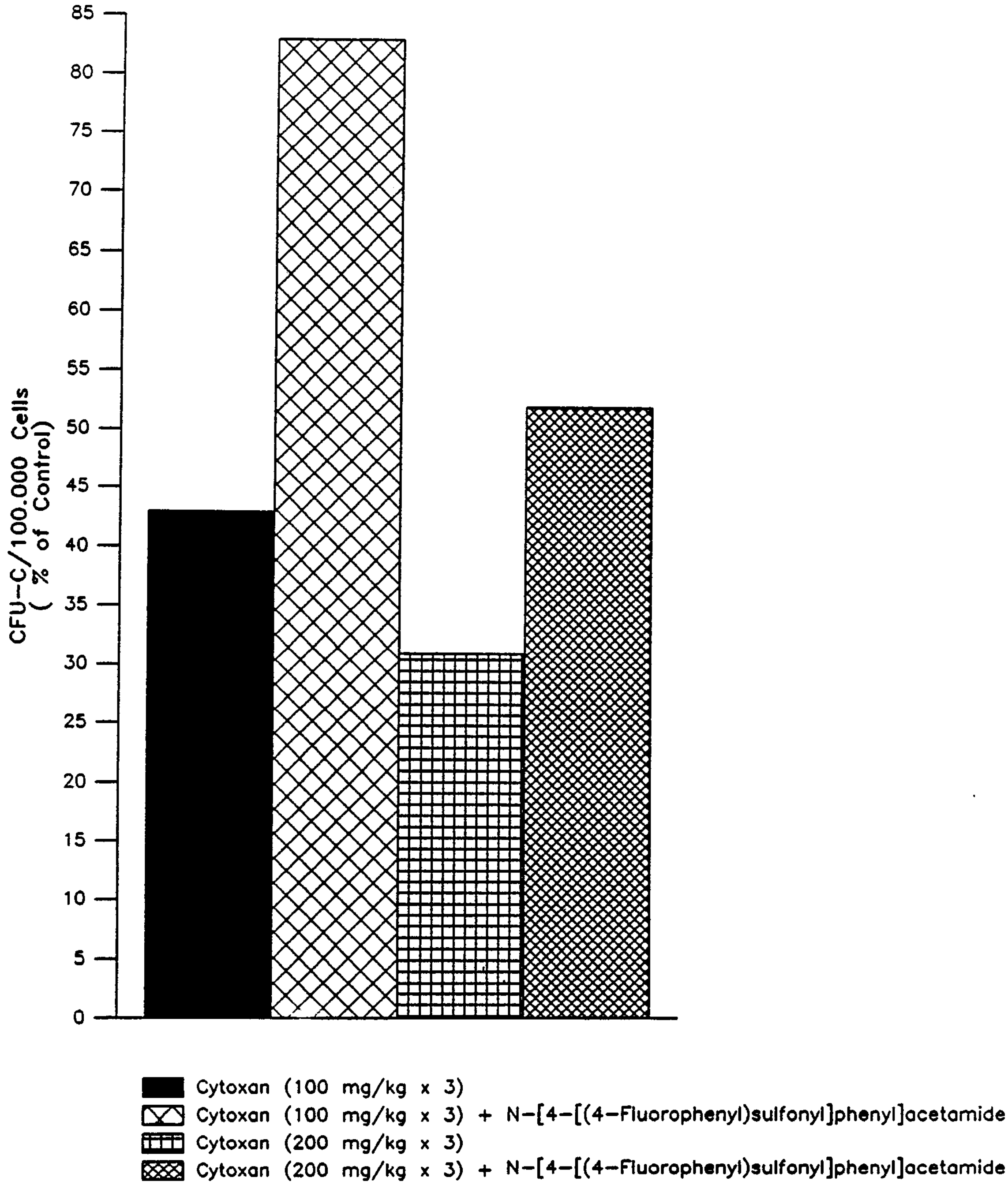

Figure 5

Effect of N-[4-[(4-Fluorophenyl)sulfonyl]phenyl]acetamide on Recovery of 5-FU Induced Neutropenia in Athymic Mice

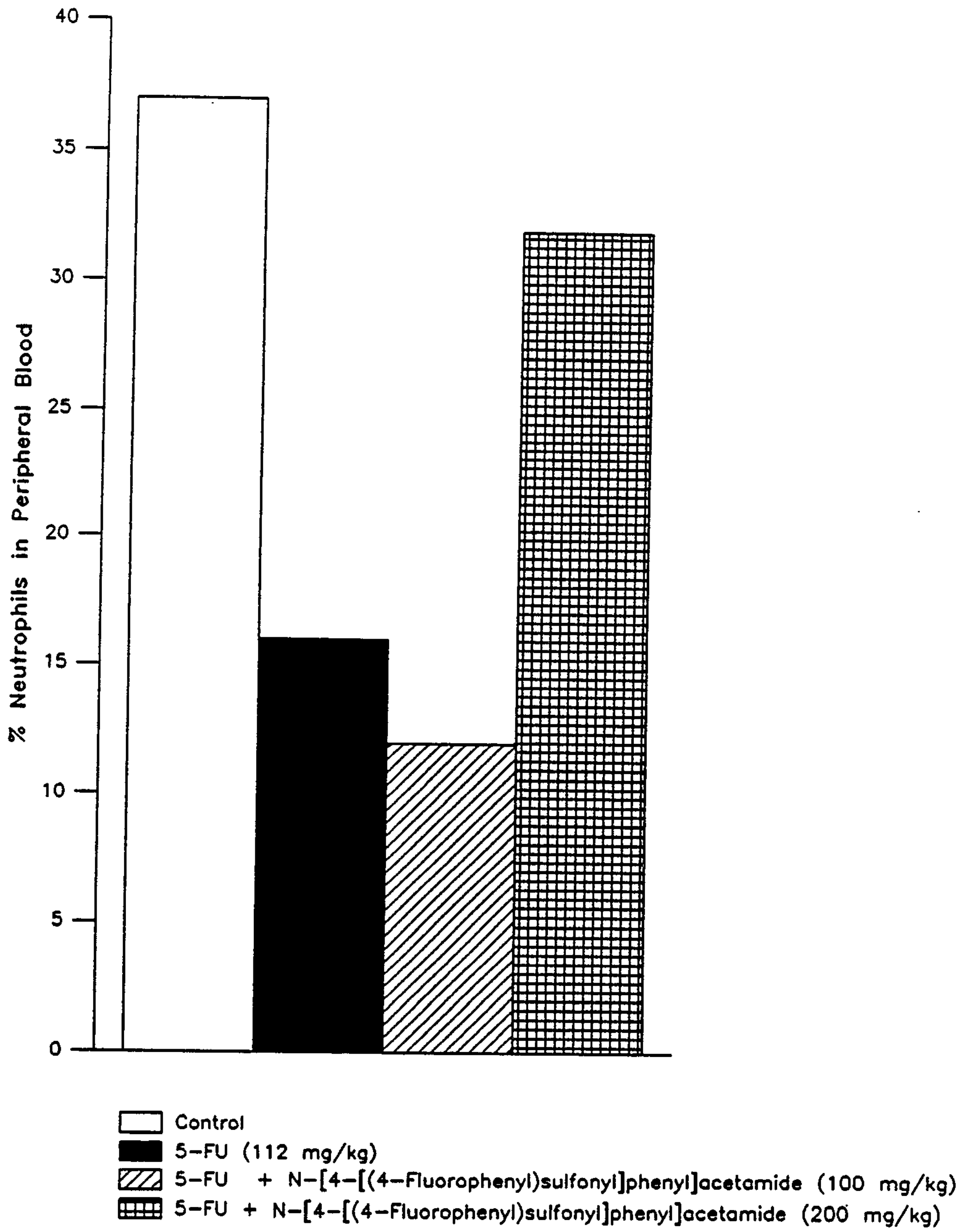

Figure 6

Effect of N-[4-[(4-Fluorophenyl)sulfonyl]phenyl]acetamide on Platelet Counts in 5-FU Treated Mice

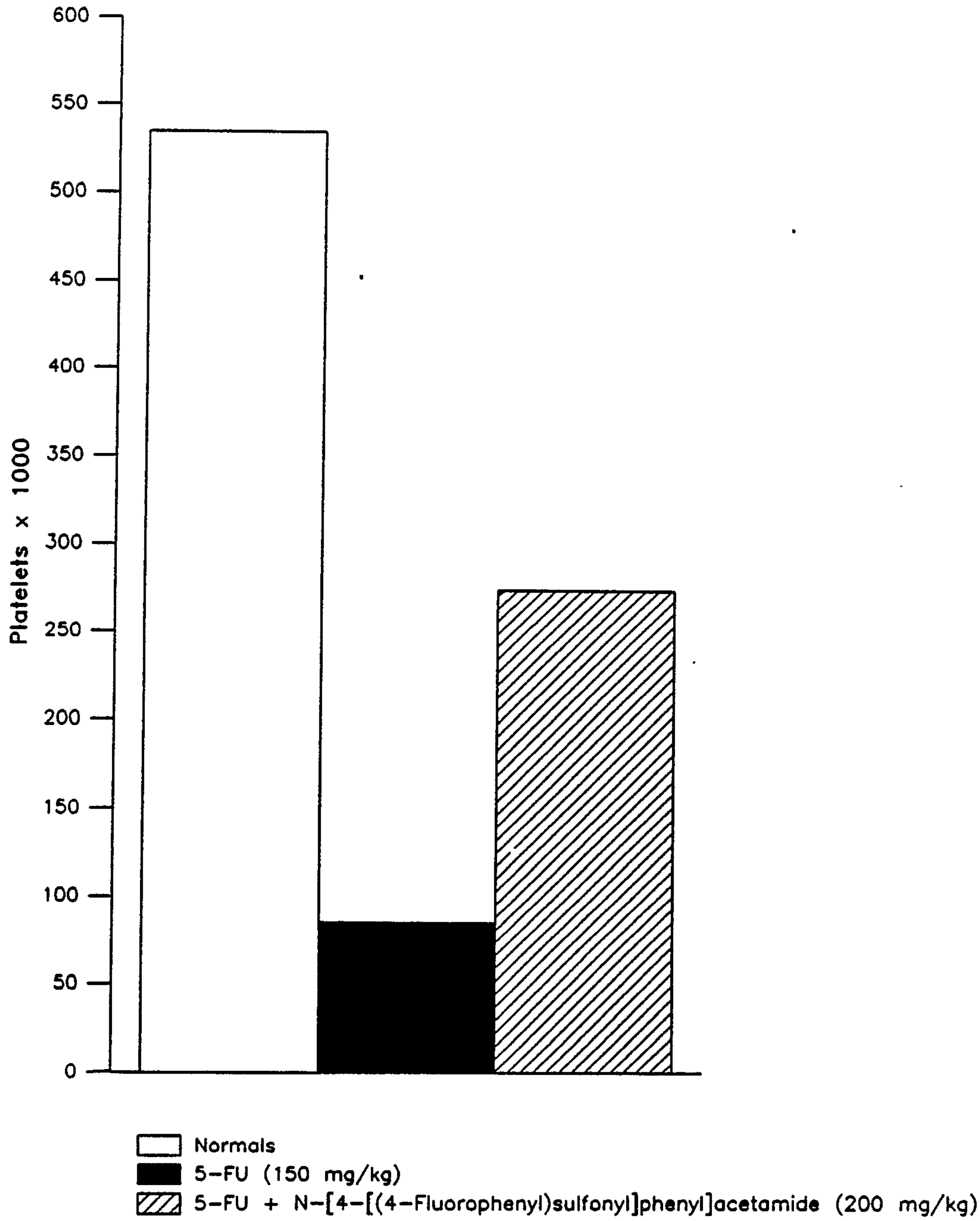

Figure 7

Effect of N-[4-[(4-Fluorophenyl)sulfonyl]phenyl]acetamide on Reticulocyte Counts in 5-FU Treated Mice

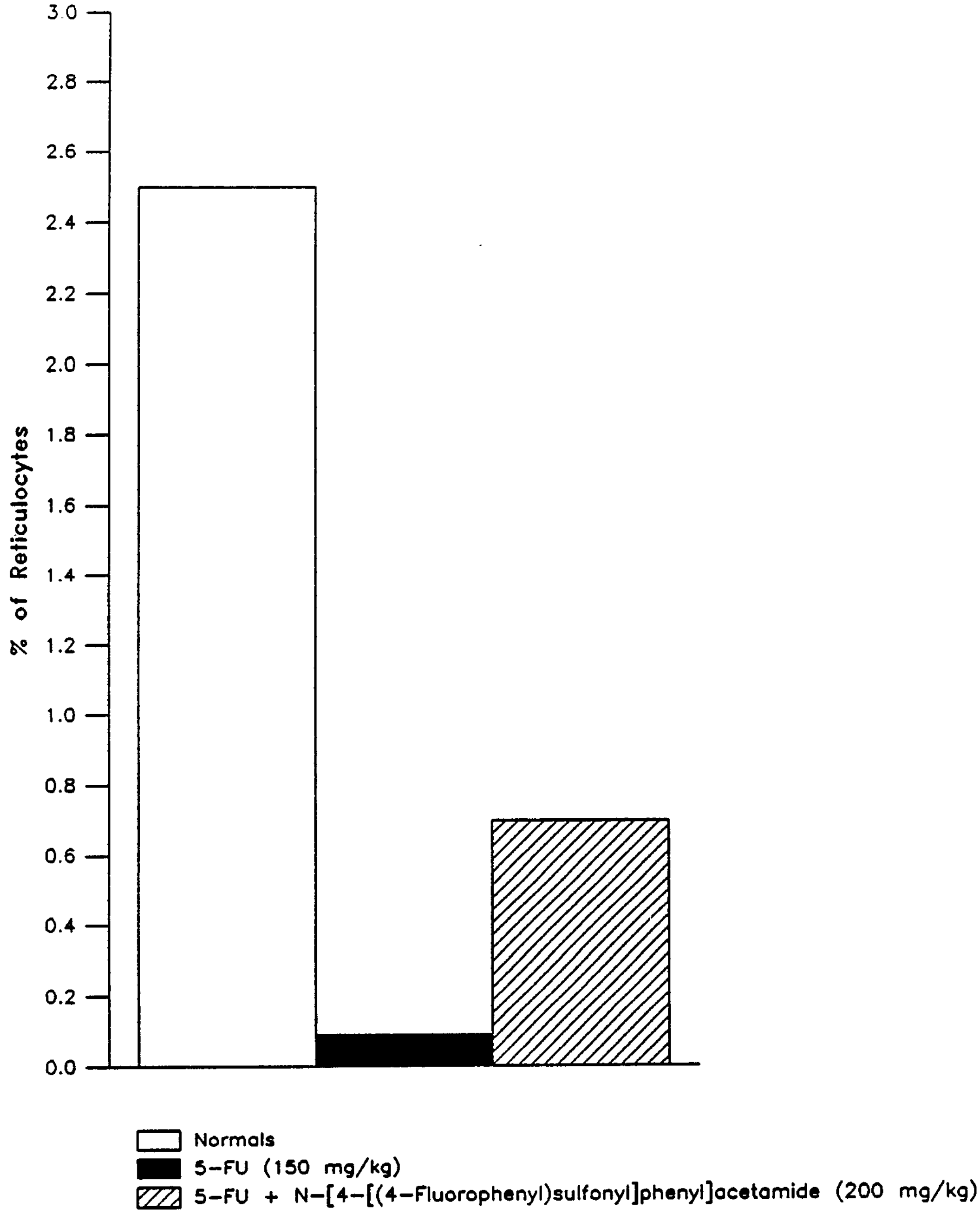

Figure 8

Effect of N-[4-[(4-Fluorophenyl)sulfonyl]phenyl]acetamide on Bone Marrow Cell Recovery After Gamma Irradiation

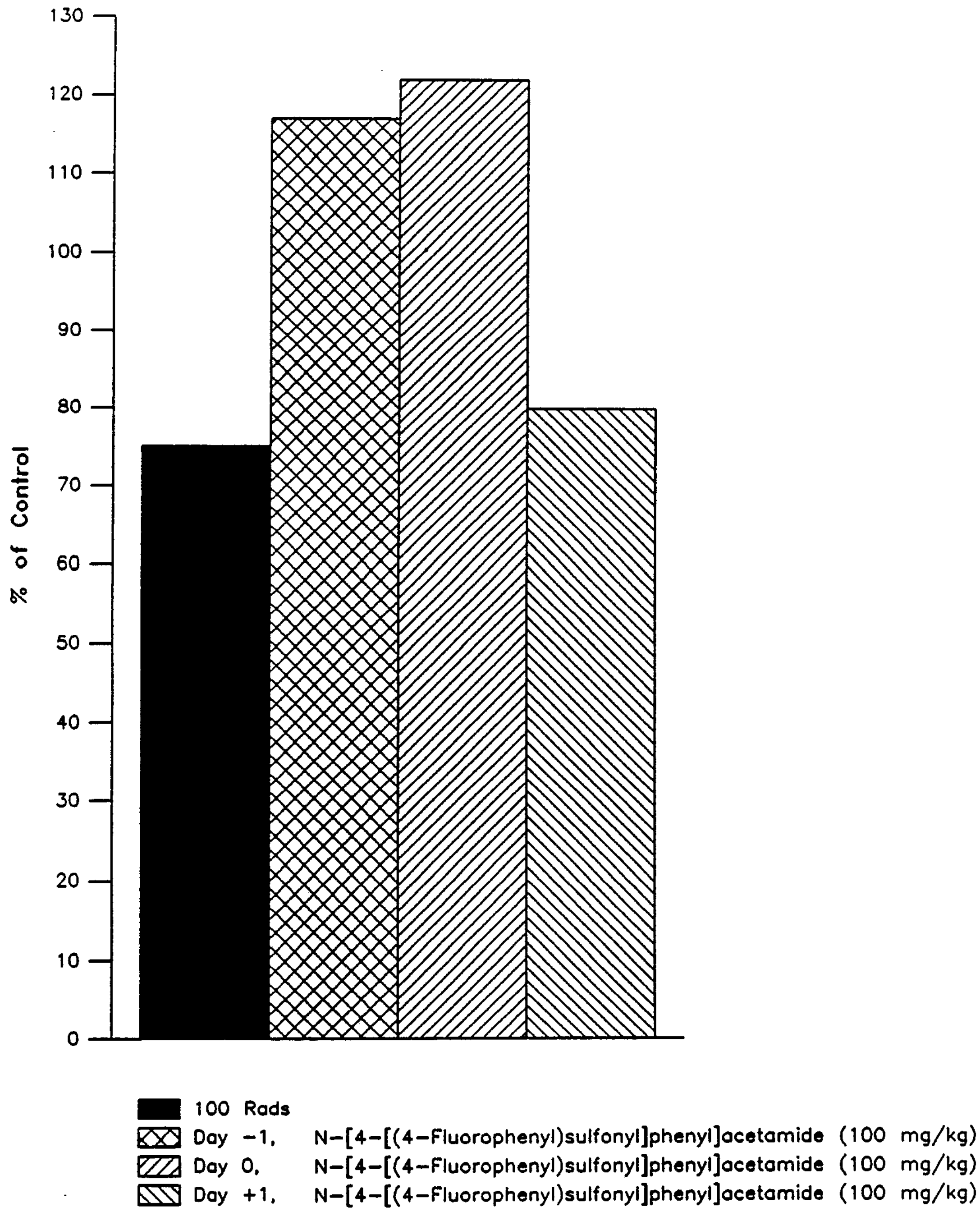

Figure 9

Effect of N–[4–[(4–Fluorophenyl)sulfonyl]phenyl]acetamide on Endogenous CFU–S Following 600 Rads of Gamma Irradiation

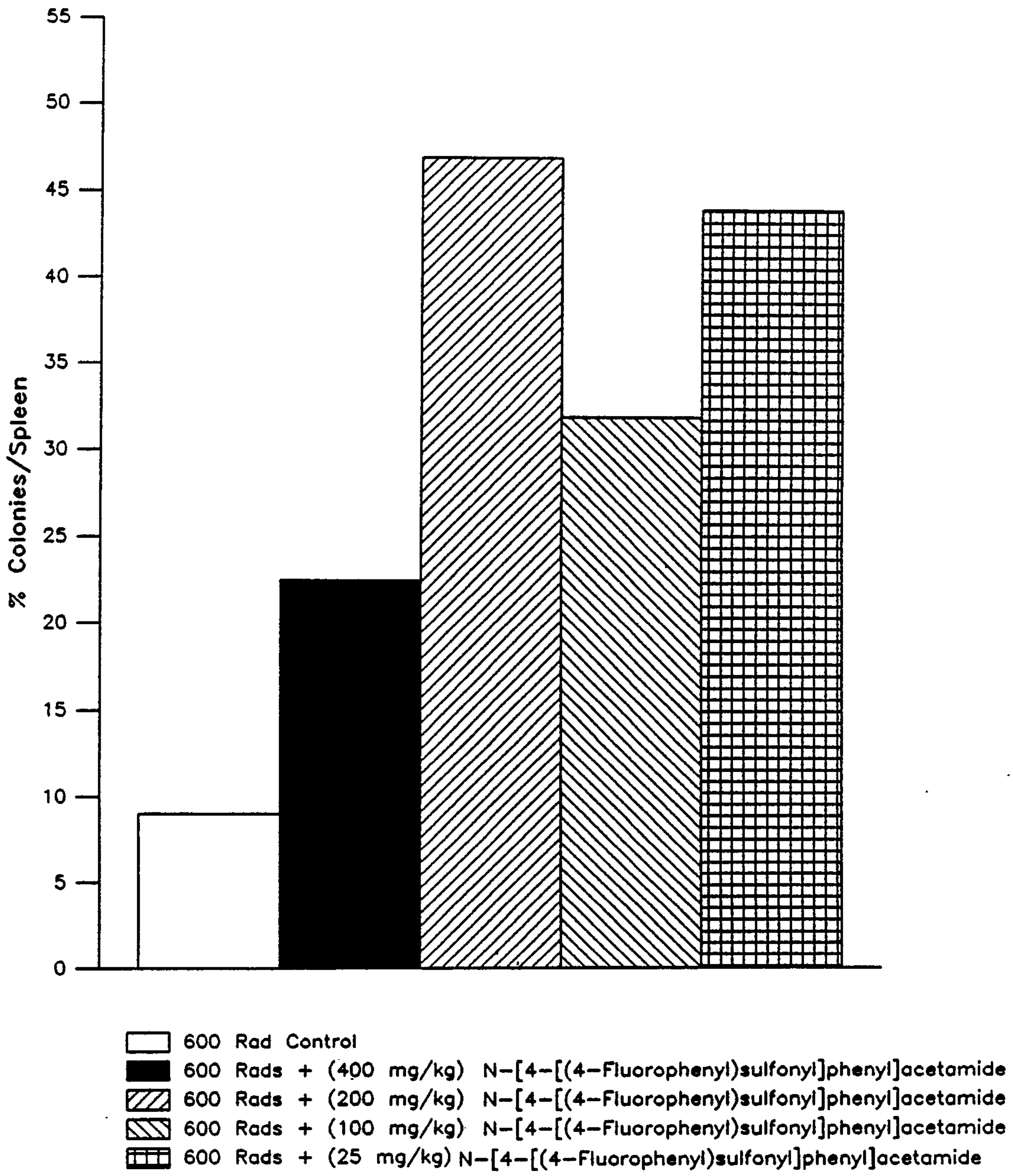

FIGURE 10

ENHANCEMENT OF IL-1 PRODUCTION

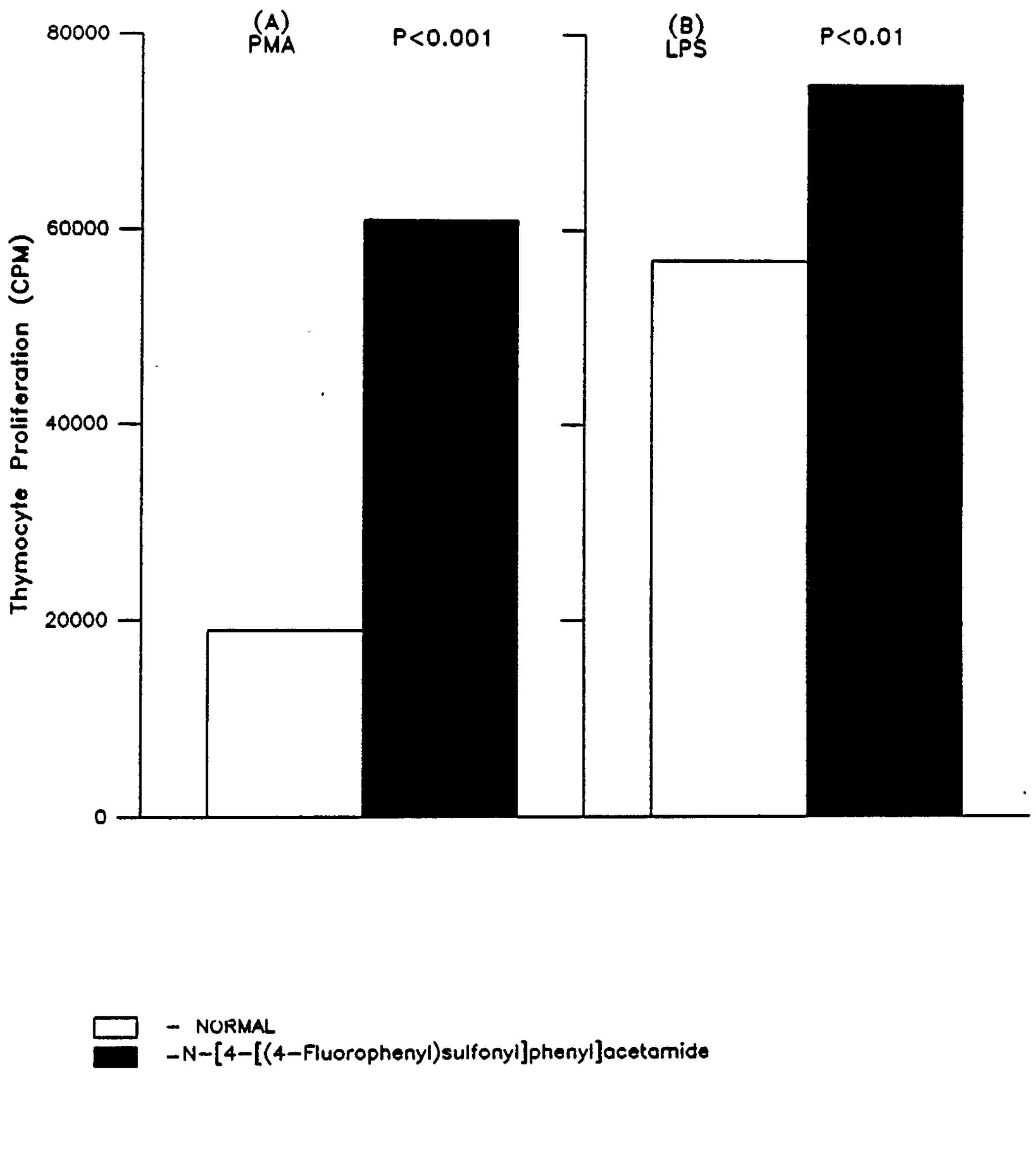

European Patent Office

# PARTIAL EUROPEAN SEARCH REPORT

which under Rule 45 of the European Patent Convention shall be considered, for the purposes of subsequent proceedings, as the European search report

Application number

EP 89 10 7999

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| X | RECENT ADV. CHEMOTHER., PROC. INT. CONGR. CHEMOTHER., 14th, vol. Anticancer Sect. 2, 1985, pages 922-3<br>F.E. DURR et al.: "Immunoprotective and immunorestorative effects of a new immunomodular, CL 259, 763"<br>* The whole document *<br>-- | 9,10 | A 61 K 31/135<br>A 61 K 31/275<br>A 61 K 31/41<br>A 61 K 31/165 |
| A | EP-A-0 102 476 (AMERICAN CYANAMID CO.)<br>* Claim 1; page 16, lines 1-19 *<br>-- | 9,10 | |
| A | FR-A-2 259 596 (I.C.I.)<br>* Claim 1 *<br>-- ./. | 9,10 | |

TECHNICAL FIELDS SEARCHED (Int. Cl.4)

A 61 K

## INCOMPLETE SEARCH

The Search Division considers that the present European patent application does not comply with the provisions of the European Patent Convention to such an extent that it is not possible to carry out a meaningful search into the state of the art on the basis of some of the claims.

Claims searched completely: 9,10

Claims searched incompletely:

Claims not searched: 1-8

Reason for the limitation of the search:

Method for treatment of the human or animal body by surgery or therapy (see Art. 52(4) of the European Patent Convention).

| Place of search | Date of completion of the search | Examiner |
|---|---|---|
| The Hague | 09-07-1990 | GERLI |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1505.1 03.82

European Patent Office

PARTIAL EUROPEAN SEARCH REPORT

Application number EP 89 10 7999 -2-

| DOCUMENTS CONSIDERED TO BE RELEVANT | | | CLASSIFICATION OF THE APPLICATION (Int. Cl.4) |
|---|---|---|---|
| Category | Citation of document with indication, where appropriate, of relevant passages | Relevant to claim | |
| A | BIOCHEMICAL PHARMACOL. vol. 32, no. 2, 1983, pages 361-6, Pergamon Press Ltd., GB; R.J. BONNEY et al.: "Inhibition of the release of prostaglandins. Leukotrienes and lysosomal acid hydrolases from macrophages by selective inhibitors of lecithin biosynthesis" | | |
| | * The whole document * | 9,10 | |
| | -- | | TECHNICAL FIELDS SEARCHED (Int Cl.4) |
| A | ARZNEIM.-FORSCH. vol. 36, no. 2, 1986, pages 230-3; H. KÖLLING et al.: "Untersuchungen an malariawirksamen ω-Aminoacyl-Verbindungen" | | |
| | * The whole document * | 9,10 | |
| | -- | | |
| X | CANCER IMMUNOL IMMUNNOTHER, vol. 22, 1986, pages 8-14, Cancer Immunology Immunotherapy, Springer Verlag; B.S. WANG et al.: "Modulation of the immune response to tumors by a novel synthetic compound, N-(14-((4-fluorophenyl)sulfonyl)phenyl) acetamide (CL 259,763)" | | |
| | * Page 12, left-hand column, lines 18-23, table 5 * | 9,10 | |
| | -- | | |
| X | CANCER RESEARCH, vol. 48, April 15, 1988, pages 2135-7; B.S. WANG et al.: "Restoration of Cytolitic T-lymphocyte response with a new immunopetentiator, N-(4-((4-fluorophenyl)sulfonyl)phenyl) acetamide (CL 259,763), in mice" | | |
| | * Page 2137, right-hand column, lines 7-21 * | 9,10 | |
| | ------------ | | |